# EUROPÄISCHE PATENTSCHRIFT

(11) **EP 3 097 901 B1**
(45) Veröffentlichungstag und Bekanntmachung des Hinweises auf die Patenterteilung: **17.01.2018**
(21) Anmeldenummer: 16168442.8
(22) Anmeldetag: 04.05.2016
(51) Int. Cl.: A61J 1/06, A61J 7/00, A61J 1/14, A61M 5/28, B23K 26/20

(54) **BEHÄLTER ZUM LAGERN UND/ODER APPLIZIEREN EINER PHARMAZEUTISCHEN SUBSTANZ SOWIE VERFAHREN ZU SEINER HERSTELLUNG**
CONTAINER FOR STORING AND/OR APPLYING A PHARMACEUTICAL SUBSTANCE AND METHOD FOR PRODUCING THE SAME
RECIPIENT DESTINE A STOCKER ET/OU A APPLIQUER UNE SUBSTANCE PHARMACEUTIQUE ET SON PROCEDE DE PRODUCTION

(30) Priorität: 28.05.2015 DE 102015108431
(43) Veröffentlichungstag der Anmeldung: 30.11.2016
(73) Patentinhaber: Schott AG, 55122 Mainz (DE)
(72) Erfinder: Hoppe, Bernd, 55218 Ingelheim (DE); Bamberg, Klaus, 9524 Zuzwil (CH); Kücük, Mustafa, 9422 Staad (CH)
(74) Vertreter: Westphal, Mussgnug & Partner Patentanwälte mbB

(56) Entgegenhaltungen:
- EP-A1- 2 189 270
- JP-A- 2007 261 666
- US-A- 4 832 214

## Beschreibung

Die vorliegende Erfindung betrifft einen Behälter zum Lagern und/oder Applizieren einer pharmazeutischen Substanz. Unter einer pharmazeutischen Substanz soll eine Substanz wie ein Medikament verstanden werden, welches gezielt zur Behandlung des menschlichen oder tierischen Körpers verwendet wird. Die pharmazeutischen Substanzen, die im erfindungsgemäßen Behälter gelagert werden können, können sowohl pastöse, flüssige als auch gasförmige Substanzen und Gemische sowie Dispersionen und Emulsionen sein. Da Glas eine hohe Inertie gegenüber einem Großteil der gebräuchlichen pharmazeutischen Substanzen und eine hohe Diffusionsdichtigkeit aufweist, eignet es sich besonders zum Lagern von pharmazeutischen Substanzen. Aufgrund der hohen Diffusionsdichtigkeit sind Permeationsverluste während der Lagerung gering, was insbesondere bei hochwertigen pharmazeutischen Substanzen ein wichtiger Aspekt ist.

Insbesondere bei modernen pharmazeutischen Wirkstoffen, die sehr teuer, hochwirksam und sehr empfindlich sind, geht man mehr und mehr dazu über, vorgefüllte Spritzen oder Karpulen einzusetzen, wobei sich aus den oben genannten Gründen Spritzen aus Glas anbieten. Mit vorgefüllten Spritzen ist es nicht mehr notwendig, den Wirkstoff von einem Behälter in einen anderen Behälter zu überführen. Vielmehr ist die vorgefüllte Spritze sofort nach dem Entpacken gebrauchsfertig. Neben der Zeitersparnis für den Arzt oder die Krankenschwester kommt der Vorteil hinzu, dass Verluste, die beim Überführen von einem in den anderen Behälter häufig auftreten, vermieden werden. Zudem besteht beim Überführen das Risiko einer Infektion oder einer Kontamination der Substanz und/oder der Spritze. Dieses Risiko wird mit vorgefüllten Spritzen deutlich verringert.

Spritzen weisen einen Grundkörper auf, welcher eine im Wesentlichen hohlzylindrische Form aufweist, weshalb Rohrglas für den Grundkörper verwendet wird. Daneben weisen Spritzen relativ komplizierte Geometrien auf, um Kanülen oder Schläuche zum Applizieren der pharmazeutischen Substanzen anschließen zu können. Als Beispiel sei an dieser Stelle ein Luer-Lock-Anschluss erwähnt, der nur mit erheblichem Aufwand aus Glas zu fertigen ist. Besonders aufwendig ist die Fertigung eines Luer-Lock-Anschlusses oder anderer Geometrien direkt aus dem Rohrglas, wozu ein mehrstufiger Heißformgebungsprozess mit verketteten Umformprozessen durchgeführt wird. Alle Umformprozesse müssen aufeinander abgestimmt sein, da durch die Verkettung auch eine gegenseitige Beeinflussung der Umformprozesse untereinander und der so erhaltenen Formen erfolgt.

Alternativ ist es möglich, mehrere vorgefertigte Anschlusskörper aus Glas, welche bereits die gewünschte Geometrie aufweisen, mit dem Rohrglas zu verbinden. Beispielsweise können die vorgefertigten Anschlusskörper mit thermischen Fügeverfahren miteinander verbunden werden, wodurch ein Stoffschluss zwischen den Anschlusskörpern und dem Rohrglas erzeugt wird. Aufgrund des Stoffschlusses weist die so erzeugte Spritze eine hohe Diffusionsdichtigkeit auf, weshalb sie sich genauso wie die direkt aus dem Rohrglas hergestellte Spritze besonders gut für das Lagern und/oder Applizieren von pharmazeutischen Substanzen eignet. Hierzu müssen allerdings das Rohrglas und die Anschlusskörper bis zu einer Temperatur oberhalb des Transformationspunktes T_{G} erhitzt werden, bei der sie nicht mehr formstabil sind, so dass auch hier die Fertigung sehr aufwendig wird, um die Behälter mit der geforderten Präzision zu fertigen.

In der WO 96 024 73 A1 wird ein Licht-absorbierendes Material zwischen zwei Glasplatten angeordnet, wodurch diese miteinander verbunden werden können. Die WO 2014/201315 A1 zeigt ein Verfahren, bei dem ein Grundkörper aus Glas mit zwei Glasschichten dadurch verbunden, dass die Glasschichten eine höhere Strahlungsabsorption für elektromagnetische Wellen aufweisen als der Grundkörper. In der DE 10 2008 023 826 A1 wird ein erstes Bauteil mit einem zweiten Bauteil mittels eines Fügelots verbunden, wobei sowohl die Bauteile als auch das Fügelot aus Glas oder Glaskeramik bestehen und das Fügelot eine stärkere Strahlungsabsorption aufweist als die beiden Bauteile.

In der US 2010/0280414 A1 ist eine Spritze gezeigt, bei der die Anschlusskörper auf mechanischem Wege mit dem Rohrglas verbunden sind, ohne dass dabei ein Stoffschluss vorliegt. Derartige Spritzen eignen sich aber nicht für die Lagerung von pharmazeutischen Substanzen, da sie entweder aufgrund der mechanischen Verbindung nicht in ausreichendem Maße diffusionsdicht sind oder die mechanische Verbindung aufwendig abgedichtet werden muss, weshalb Dichtkörper mit der pharmazeutischen Substanz in Kontakt kommen können. In beiden Fällen bleibt das Risiko des Eindringens von Bakterien und Viren oder anderer Fremdstoffe durch die mechanische Verbindung, was zu einer Kontamination der pharmazeutischen Substanz führen kann. Ferner sind Permeationsverluste durch die Dichtkörper nicht ausgeschlossen, was bei den häufig teuren pharmazeutischen Substanzen ein großer Nachteil ist.

Aufgabe der vorliegenden Erfindung ist es daher, einen Behälter zum Lagern und/oder Applizieren einer pharmazeutischen Substanz anzugeben, der eine hohe Diffusionsdichtigkeit aufweist, die Permeationsverluste in engen Grenzen hält und einfach zu fertigen ist.

Gelöst wird die Aufgabe mit einem Behälter nach Anspruch 1 und Anspruch 10. Vorteilhafte Ausgestaltungen des erfindungsgemäßen Behälters ergeben sich aus den Unteransprüchen.

Der erfindungsgemäße Behälter zum Lagern und/oder Applizieren einer pharmazeutischen Substanz umfasst einen Grundkörper aus Glas, der eine im Wesentlichen hohlzylindrische Form aufweist und einen Hohlraum umschließt, wobei der Grundkörper ein erstes Ende mit einer ersten Öffnung aufweist, und einen ersten Anschlusskörper aus Glas, wobei der erste Anschlusskörper einen mit der ersten Öffnung kommunizierenden Durchtrittskanal aufweist, der erste Anschlusskörper in einem ersten Anschlussbereich mit dem Grundkörper verbunden ist und der Behälter im ersten Anschlussbereich eine oder mehrere erste Absorptionszonen aufweist, in welcher der Behälter eine höhere Strahlungsabsorption für elektromagnetische Wellen in einem vorbestimmten Wellenlängenbereich aufweist als der Grundkörper außerhalb der ersten Absorptionszone.

Der erste Anschlusskörper schließt sich entweder direkt oder indirekt dem ersten Ende des Grundkörpers an. Definitionsgemäß soll der erste Anschlussbereich den Bereich der Kontaktfläche des Grundkörpers umfassen, über die der Grundkörper entweder direkt oder indirekt mit dem ersten Anschlusskörper in Kontakt tritt, kann sich dabei aber auch noch etwas zum Zentrum des Grundkörpers erstrecken, wobei die Erstreckung so gering wie technisch möglich gehalten werden soll. Davon ausgehend soll der erste Anschlussbereich den gesamten Anschlusskörper umfassen. In diesem ersten Anschlussbereich befindet sich die Absorptionszone, in welcher der Behälter eine höhere Strahlungsabsorption für elektromagnetische Wellen in einem vorbestimmten Wellenlängenbereich aufweist als der Grundkörper außerhalb der ersten Absorptionszone. Die erste Absorptionszone wird innerhalb des ersten Anschlussbereichs so gelegt, dass der Grundkörper entweder direkt oder indirekt mit dem Anschlusskörper verbunden werden kann. Je nach Ausgestaltung des Behälters kann die erste Absorptionszone auf das erste Ende des Grundkörpers begrenzt sein. In diesem Fall hat im Wesentlichen nur die Kontaktfläche des Grundkörpers, an welcher der Grundkörper mit dem ersten Anschlusskörper direkt oder indirekt verbunden ist, eine höhere Strahlungsabsorption für elektromagnetische Wellen. Alternativ kann sich die erste Absorptionszone über den Bereich des ersten Endes des Grundkörpers inklusive der Kontaktfläche erstrecken. Ebenfalls ist es denkbar, dass sich die erste Absorptionszone ganz oder teilweise über den ersten Anschlusskörper erstreckt, wobei die mit der Kontaktfläche zusammenwirkende Fläche des ersten Anschlusskörpers eingeschlossen ist. Wichtig ist, dass sich die erste Absorptionszone nicht über den gesamten Grundkörper erstreckt sondern gar nicht oder nur teilweise. Andere, hier nicht aufgezählte Konstellationen sind ebenfalls eingeschlossen.

Als Strahlungsquellen zum Erzeugen der elektromagnetischen Wellen seinen beispielhaft Quecksilberdampflampen, die UV-Strahlen erzeugen, Xenon-Kurzbogen-Hochdrucklampen, die sichtbare Lichtstrahlen erzeugen, Infrarot-Strahlungsquellen wie z.B. ein Nd:YAG-Laser, ein Diodenlaser oder ein Wolfram-IR-Strahler, oder ein Magnetron zum Erzeugen von Mikrowellen genannt. Die Ausgestaltung des Behälters und insbesondere der Absorptionszonen wird dabei unter Berücksichtigung der verwendeten Strahlungsquellen vorgenommen. Hierbei ist man bestrebt, den vorbestimmten Wellenlängenbereich so eng wie technisch möglich zu wählen, so dass vorzugsweise nur eine Wellenlänge verwendet wird, wie es mit Lasern besonders gut machbar ist.

Der Behälter weist in der ersten Absorptionszone eine höhere Strahlungsabsorption auf als der Grundkörper außerhalb der ersten Absorptionszone und folglich auch außerhalb des ersten Anschlussbereichs. Es ist somit möglich, beim Einwirken von elektromagnetischen Wellen den Grundkörper und/oder den Anschlusskörper in der ersten Absorptionszone gezielt lokal höher zu erhitzen als außerhalb der ersten Absorptionszone, in welchem der Behälter eine niedrigere Strahlungsabsorption aufweist. Zumindest ein Teil des Grundkörpers weist eine niedrigere Strahlungsabsorption auf. Generell kann eine erhöhte Strahlungsabsorption durch eine Erhöhung des Absorptionskoeffizienten und/oder durch eine Erhöhung der Weglänge der Strahlung in der ersten Absorptionszone herbeigeführt werden. Hierdurch werden der Grundkörper und/oder der Anschlusskörper nur im Bereich der Fügestelle bzw. der ersten Kontaktfläche über den Transformationspunkt hinaus erhitzt, so dass sie unter Ausbildung eines Stoffschlusses miteinander verbunden werden. Der übrige Bereich wird weniger stark erhitzt, so dass dieser Bereich formstabil bleibt, wodurch keine Dimensions- oder Formänderungen auftreten, was für die präzise Fertigung ein großer Vorteil ist.

In einer weiteren Ausführungsform ist im ersten Anschlussbereich ein erster Verbindungskörper aus Glas angeordnet, über welchen der erste Anschlusskörper mit dem Grundkörper verbunden ist. In dieser Ausführungsform wird ein Verbindungskörper zum Verbinden des ersten Anschlusskörpers mit dem Grundkörper verwendet, der zwischen dem ersten Anschlusskörper und dem Grundkörper angeordnet ist. Die hiermit erzielbaren Vorteile und technischen Effekte entsprechen denjenigen, die für den zuvor beschriebenen Behälter genannt worden sind. Die erste Absorptionszone muss sich dabei nicht auf den ersten Verbindungskörper erstrecken. Es genügt, wenn sich die erste Absorptionszone über den Bereich des ersten Endes des Grundkörpers und ganz oder teilweise über den ersten Anschlusskörper erstreckt. In diesem Fall teilt der erste Verbindungskörper die erste Absorptionszone in zwei Teile, so dass mehrere erste Absorptionszonen vorhanden sind. Diese Ausführungsform bietet sich beispielsweise an, um Durchmesserunterschiede zwischen dem Grundkörper und dem ersten Anschlusskörper zu überbrücken.

In einer weiteren Ausgestaltung ist die erste Absorptionszone auf den ersten Verbindungskörper begrenzt. Mit anderen Worten wird der erste Anschlussbereich ausschließlich vom ersten Verbindungskörper gebildet. Folglich weist nur der erste Verbindungskörper eine erhöhte Strahlungsabsorption auf, so dass der Grundkörper und der Anschlusskörper völlig unverändert bleiben können, um sie auf die erfindungsgemäße Weise miteinander zu verbinden. Dabei muss sich die erste Absorptionszone nicht vollständig über den ersten Verbindungskörper erstrecken. Es genügt, wenn der erste Verbindungskörper an den Kontaktflächen oder den Fügestellen zum Anschlusskörper und zum Grundkörper eine erhöhte Strahlungsabsorption aufweist. In diesem Fall sind zwei erste Absorptionszonen vorhanden. Hierdurch kann der erfindungsgemäße Behälter besonders kostengünstig und einfach gefertigt werden.

In einer weiteren Ausgestaltung umfasst der Behälter einen zweiten Anschlusskörper aus Glas. Zudem weist der Grundkörper ein zweites Ende mit einer zweiten Öffnung auf, wobei der zweite Anschlusskörper in einem zweiten Anschlussbereich mit dem Grundkörper verbunden ist und der Behälter im zweiten Anschlussbereich eine oder mehrere zweite Absorptionszonen aufweist, in welcher der Behälter zumindest abschnittsweise eine höhere Strahlungsabsorption für elektromagnetische Wellen in einem vorbestimmten Wellenlängenbereich aufweist als der Grundkörper außerhalb der zweiten Absorptionszone.

Die für den Behälter, der nur den ersten Anschlusskörper aufweist, genannten Vorteile gelten für diese Ausgestaltung genauso. Insbesondere eignet sich diese Ausgestaltung des erfindungsgemäßen Behälters zum Bereitstellen von Spritzen zum Applizieren der pharmazeutischen Substanzen, beispielsweise in den menschlichen oder tierischen Körper, da der erste Anschlusskörper beispielsweise als ein Luer-Lock-Anschluss und der zweite Anschlusskörper als ein Fingerflansch ausgebildet sein können.

Luer-Lock-Anschlüsse finden in labortechnischen, medizinischen und pharmazeutischen Anwendungen weite Verbreitung, beispielsweise, um Schläuche oder Kanülen am ersten Ende anzuschließen. Ein Luer-Lock-Anschluss ist ein genormtes Bauteil, welches im Wesentlichen ein Innengewinde mit einer genormten, relativ großen Steigung, und einen koaxial verlaufenden Kegel umfasst. Da der Luer-Lock-Anschluss normgerecht gefertigt werden muss, werden an seine Herstellung hohe Anforderungen bezüglich der Präzision gestellt, was sich erfindungsgemäß mit der Möglichkeit der lokalen Erwärmung dort, wo der erste Anschlusskörper eine erhöhte Strahlungsabsorption für elektromagnetische Wellen aufweist, gut realisieren lässt.

Über die zweite Öffnung des zweiten Endes, an welchem der Fingerflansch angeordnet ist, kann ein Kolben in den hohlzylindrischen Grundkörper eingebracht werden kann. Der Kolben ist so gefertigt, dass er den Hohlraum gegenüber dem betreffenden zweiten Ende abdichtet, so dass keine Substanz über dieses Ende entweichen kann. In den Luer-Lock-Anschluss können geeignete Verschlüsse eingeschraubt werden, so dass der Behälter auch am ersten Ende abgedichtet ist, um den Austritt der Substanz aus dem Hohlraum zu verhindern. Nach Öffnen des Luer-Lock-Anschlusses kann eine Kanüle angeschlossen werden, so dass die Substanz bequem appliziert werden kann, wozu der Benutzer den Kolben mit dem Daumen in den Hohlraum schieben kann, während sich seine Finger am Fingerflansch abstützen. Der Fingerflansch kann auch eine "Backstop-Funktion" aufweisen, so dass der Kolben nicht versehentlich aus dem Hohlraum entfernt werden kann. Erfindungsgemäß lassen sich derartige vorfüllbare Spritzen einfach und kostengünstig fertigen.

In einer weiteren Ausgestaltung ist im zweiten Anschlussbereich ein zweiter Verbindungskörper aus Glas angeordnet, über welchen der zweite Anschlusskörper mit dem Grundkörper verbunden ist. Wie bereits zum ersten Verbindungskörper ausgeführt, eignet sich diese Ausgestaltung besonders, um Durchmesser- oder Formunterschiede zwischen dem Grundkörper und dem zweiten Anschlusskörper zu überbrücken, was zu einer flexibleren Fertigung führt.

Dabei kann die zweite Absorptionszone auf den zweiten Verbindungskörper begrenzt sein. Auch hier gilt, dass der zweite Anschlusskörper leichter mit dem Grundkörper verbunden werden kann, da es möglich ist, nur den zweiten Verbindungskörper mit einer erhöhten Strahlungsabsorption für elektromagnetische Strahlung auszustatten. Wiederum genügt es, wenn die Kontaktflächen oder der Bereich der Fügestellen eine erhöhte Strahlungsabsorption aufweisen, so dass auch mehrere zweite Absorptionszonen im Verbindungskörper vorgesehen werden können.

Zusammenfassend ist es erfindungsgemäß folglich möglich, die Absorptionszonen wie folgt anzuordnen:

Für den Fall, dass der Behälter keine Verbindungskörper umfasst, sind die Absorptionszonen
- auf dem Grundkörper im Bereich der Kontaktflächen, über welche der Grundkörper mit dem oder den Anschlusskörpern zusammenwirkt, und/oder
- auf dem oder den Anschlusskörpern zumindest im Bereich der Kontaktflächen, über welche der oder die Anschlusskörper mit dem Grundkörper zusammenwirken, angeordnet.

Für den Fall, dass der Behälter einen oder mehrere zwischen dem Grundkörper und dem Anschlusskörper angeordnete Verbindungskörper aufweist, sind die Absorptionszonen
- auf dem Grundkörper im Bereich der Kontaktflächen, über die der Grundkörper mit dem oder den Verbindungskörpern zusammenwirken,
- auf dem oder den Anschlusskörpern im Bereich der Kontaktflächen, über welche der oder die Anschlusskörper mit dem oder den Verbindungskörpern zusammenwirken, und/oder
- auf dem oder den Verbindungskörpern zumindest im Bereich der Kontaktflächen, über welche der oder die Verbindungskörper mit dem Grundkörper und dem Anschlusskörper zusammenwirken, angeordnet sind.

Es ist bevorzugt, wenn der Grundkörper, der erste Anschlusskörper, der zweite Anschlusskörper, der erste Verbindungskörper und/oder der zweite Verbindungskörper aus demselben Grundglas bestehen. Der Begriff Grundglas, auch Glastyp genannt, soll so verstanden werden, dass zwei Gläser zum selben Grundglas gehören, wenn die Zusammensetzung der Hauptkomponenten und deren Konzentrationen sowie die chemischen und physikalischen Eigenschaften im Wesentlichen gleich sind, auch wenn das eine Glas mit Fremdatomen dotiert sein sollte und das andere nicht.

Insbesondere dann, wenn der gesamte Behälter bzw. alle Körper aus demselben Grundglas hergestellt sind, hat der erfindungsgemäße Behälter dieselben Eigenschaften wie ein direkt aus dem Rohrglas hergestellter Behälter. Es sind keine Materialmodifikationen notwendig, was insbesondere für die Lagerung von pharmazeutischen Substanzen einen erheblichen Vorteil darstellt, da für den gesamten Behälter ein zugelassenes Grundglas verwendet werden kann, was die Zulassung des erfindungsgemäßen Behälters für die Lagerung von pharmazeutischen Substanzen deutlich vereinfacht. Zudem werden die Glasbeschaffung und die Lagerung des Grundglases bzw. der Grundkörper und der Anschlusskörper vereinfacht, da nicht nach unterschiedlichen Glassorten unterschieden werden muss. Zusammenfassend lässt sich der erfindungsgemäße Behälter auf einfache Weise mit einer hohen Toleranz und Formstabilität aus ein und demselben, für die Lagerung von pharmazeutischen Substanzen zugelassenem Glas herstellen.

Weiterhin ist es bevorzugt, wenn der Behälter in der oder den ersten Absorptionszonen und/oder der oder den zweiten Absorptionszonen aus Sinterglas besteht. Hierzu können beispielsweise der erste und/oder der zweite Anschlusskörper und/oder der erste und/oder der zweite Verbindungskörper aus Sinterglas bestehen. Hierbei werden beispielsweise die Anschlusskörper oder die Verbindungskörper aus Glasgries oder Glaspulver durch Pressen und Erhitzen hergestellt. Hierdurch weisen die Anschlusskörper oder die Verbindungskörper eine andere Porosität auf als der Grundkörper. Durch die Reflexion der elektromagnetischen Wellen an den Glaspartikeln verlängert sich die Weglänge, welche die elektromagnetischen Wellen beim Durchgang durch die aus Sinterglas hergestellten Anschlusskörper oder Verbindungskörper im Vergleich zum Grundkörper zurücklegen muss. Zudem erhöht sich die Streuung, weshalb sich die Strahlungsabsorption für entsprechend gewählte elektromagnetische Wellen erhöht. Die Streuung ist abhängig von der Wellenlänge, so dass die Porosität und die Streuflächen (Wände von eingeschlossenen Luftblasen, Partikelgrenzflächen) auf die verwendete Wellenlänge abgestimmt sein müssen. Die Porosität und die Streuflächen lassen sich insbesondere über die Partikelgröße des Glasgries oder des Glaspulvers einstellen.

In einer weiteren Ausführungsform umfasst das Sinterglas Primärpartikel mit einem Durchmesser D50 zwischen 0,1 µm und 200 µm. Der Durchmesser D50 besagt, dass 50% aller Primärpartikel einen Durchmesser haben, der größer als der angegebene Wert für D50 ist. In diesem Größenbereich lässt sich einerseits die Strahlungsabsorption der bevorzugt verwendeten elektromagnetischen Strahlung (sichtbares Licht, Infrarotstrahlung) wirkungsvoll erhöhen, andererseits lassen sich die Anschlusskörper, deren Sinterglas Primärpartikel in diesem Durchmesserbereich aufweist, besonders gut pressen. Die geschlossene Porosität liegt dabei vorzugsweise bei 0 bis 50%. Die geschlossene Porosität berücksichtigt dabei nur in sich geschlossene Hohlräume.

Spritzen weisen dort, wo die Kanüle angeschlossen wird, üblicherweise einen dünnen Durchtrittskanal auf. Bei Glasspritzen, die direkt aus dem Rohrglas gefertigt werden, wird dieser dünne Kanal unter Verwendung eines Wolframstifts gefertigt, der während des Umformprozesses als Formwerkzeug dient. Das erhitzte Glas wird im Bereich des Kanals auf die Mantelfläche des Wolframstifts gedrückt. Nach Abschluss des Umformprozesses wird der Wolframstift aus der Spritze entnommen und es bleibt der Kanal zurück.

Ohne Verwendung des Wolframstifts kann der dünne Durchtrittskanal nicht in der gewünschten Genauigkeit gefertigt werden. Zudem besteht die Gefahr, dass der Durchtrittskanal ohne die Verwendung des Wolframstifts beim Umformen verschlossen wird. Der Stift ist deshalb aus Wolfram, weil er die hohen Temperaturen, auf die das Glas beim Umformen gebracht werden muss, um die notwendige Viskosität zu erreichen, ohne wesentliche chemischen oder mechanischen Veränderungen übersteht. Nachteilig hieran ist aber, dass bei diesem Umformschritt ein Abrieb oder Abdampfungen entsteht, so dass Wolfram-Rückstände in der Spritze zurückbleiben, die in die gelagerte Substanz migrieren können. Dies ist insbesondere dann unerwünscht, wenn pharmazeutische Substanzen in der Spritze gelagert werden.

Im Gegensatz dazu kann der aus Sinterglas gefertigte Anschlusskörper ohne die Verwendung von Wolframstiften mit einem dünnen Durchtrittskanal gefertigt werden, da die Formgebung bei Raumtemperatur erfolgt, so dass hierdurch ein entscheidender Vorteil bei der Lagerung von pharmazeutischen Substanzen gegenüber aus Rohrglas gefertigten Spritzen erzielt werden kann. Zudem lassen sich auch Anschlusskörper mit einer komplexeren Geometrie durch die Verwendung von Sinterglas kostengünstig herstellen.

In einer weiteren Ausführungsform ist Behälter in der oder den ersten Absorptionszonen und/oder der oder den zweiten Absorptionszonen zum Erhöhen der Strahlungsabsorption für elektromagnetische Wellen dotiert. Hierzu können beispielsweise der erste und/oder der zweite Anschlusskörper und/oder der Grundkörper und/oder der erste und/oder der zweite Verbindungskörper zum Erhöhen der Strahlungsabsorption für elektromagnetische Wellen dotiert sein. Hierbei werden gezielt Fremdatome in die Anschlusskörper, die Verbindungskörper und/oder in den Grundkörper eingebracht, welche den Strahlungskoeffizienten und folglich die Strahlungsabsorption erhöhen. Die dabei verwendete Konzentration an Fremdatomen bewegt sich in etwa im Bereich zwischen 0,1% und 5%. In dieser Konzentration wird die Strahlungsabsorption erhöht, ohne jedoch die Eigenschaften des Glases selbst in einem nennenswerten Umfang zu ändern. Folglich weist das dotierte Glas mit Ausnahme der Strahlungsabsorption dieselben chemischen und physikalischen Eigenschaften auf wie das nicht dotierte Glas, so dass sich die Dotierung nicht negativ auf die Fertigung des Behälters und die Lagerung der pharmazeutischen Substanzen auswirkt. Es handelt sich also um dasselbe Grundglas. Folglich entsteht ein Behälter, welcher überall dieselben Eigenschaften aufweist. Insbesondere vorteilhaft können der erste und/oder der zweite Anschlusskörper mit Verbindungen von Chrom, Nickel, Kupfer, Eisen, Kobalt, seltene Erden (z.B. Ytterbium, Dysprosium) oder andere im interessierenden Wellenlängenbereich absorbierenden Elementen, Materialien oder Verbindungen dotiert sein. Bei der Verwendung von Eisen für die Dotierung kann jegliches Eisenoxid verwendet werden, da sich im Glas ein Redox-Gleichgewicht zwischen Eisen-(II)-oxid und Eisen-(III)-oxid einstellt. Auch Kombinationen der vorgenannten Verbindungen sind möglich. Bei der Verwendung von Sinterglas kann die Dotierung dadurch vorgenommen werden, dass das die Strahlungsabsorption erhöhende Material in der gewünschten Konzentration zugemischt wird.

Einige der oben genannten Materialien bewirken beim Dotieren eine Verfärbung des dotierten Glases. So bewirkt beispielsweise Eisen eine Verdunkelung oder eine Braunverfärbung des dotierten Glases. Die Verdunkelung oder die Verfärbung kann dazu nutzen, die Behälter mit einer Markierung zu versehen und hierdurch eine optische Unterscheidung zu bewirken. Mit der optischen Unterscheidung kann sichergestellt werden, dass eine pharmazeutische Substanz nur in einen Behälter mit einer bestimmten farblichen Markierung abgefüllt wird. Zudem kann das Verwechslungsrisiko für Ärzte und Krankenschwestern beim Applizieren hierdurch verringert werden.

Bei der Verwendung von Sinterglas können die zum Dotieren verwendeten Materialien eine ganz andere Verfärbung hervorrufen als bei Glas, welches aus dotiertem Bulk-Glas hergestellt ist. Sinterkörper, die aus Glaspulver aus dotiertem Bulk-Glas hergestellt sind, erscheinen hellgrau, fast weiß, so dass der gesinterte Körper sehr hell ist, was ebenfalls zu Markierungszwecken genutzt werden kann.

In einer besonderen Ausgestaltung werden der erste und/oder der zweite Anschlusskörper und/oder der Grundkörper und/oder der erste und/oder der zweite Verbindungskörper von mehrphasigem Sinterglas gebildet. Die Strahlungsabsorption des aus dem Sinterglas gebildeten Körpers lässt sich durch den Anteil der die Strahlungsabsorption erhöhende Phase genau einstellen. Dies geschieht dadurch, dass man lokal eine deutlich höhere Konzentration von die Strahlungsabsorption erhöhendem Material einstellt, beispielsweise durch die Zumischung von keramischen Pigmenten. Hierdurch kann man auf die Dotierung verzichten, was insofern vorteilhaft ist, als dass die Konzentrationen des die Strahlungsabsorption erhöhenden Materials nicht so genau eingestellt werden müssen.

In einer weiteren Ausgestaltung kann der Grundkörper eine Fügefläche und der erste und/oder der zweite Anschlusskörper eine Gegenfügefläche aufweisen, an denen der Grundkörper mit dem ersten und/oder dem zweiten Anschlusskörper verbunden ist, wobei sich der erste und/oder der zweite Anschlusskörper im Bereich der Gegenfügefläche chemisch und/oder strukturell vom Grundkörper unterscheidet. Sinngemäß gilt dies auch für die Verbindungskörper. Die chemische Zusammensetzung und/oder die Struktur werden dort so geändert, dass die Strahlungsabsorption für elektromagnetische Wellen erhöht wird. Vorteilhaft hieran ist, dass die Strahlungsabsorption nur im Bereich der Fügeflächen und der Gegenfügeflächen erhöht ist, so dass die übrigen Bereiche des Behälters beim Fügen nicht soweit erhitzt werden, dass sie erweichen und ihre Form verlieren.

Gemäß einer weiteren Ausführungsform ist der Behälter in der oder den ersten Absorptionszonen und/oder in der oder den zweiten Absorptionszonen mit einer Diffusionsfarbe behandelt. Hierzu können der erste und/oder der zweite Anschlusskörper im Bereich der Gegenfügefläche mit einer Diffusionsfarbe behandelt sein. Alternativ oder kumulativ kann der Grundkörper im Bereich der Fügeflächen mit einer Diffusionsfarbe behandelt sein. Sinngemäß gilt dies auch für die Verbindungskörper. Diffusionsfarben sind insbesondere silberhaltige Stoffe, deren farblich wirksame Bestandteile während einer Temperaturbehandlung nach dem Auftragen auf den Grundkörper und/oder die Anschlusskörper in die anliegenden und oberen Glasschichten durch Diffusion eindringen und dort Komplexverbindungen mit dem Glas eingehen. Als Resultat werden die oberen Glasschichten je nach Zusammensetzung der Diffusionsfarben gelb-dunkelgelb bis rotbraun gefärbt, ohne dass hierdurch die mechanischen und chemischen Eigenschaften nennenswert geändert werden. Allein aufgrund der Färbung erhöht sich die Strahlungsabsorption für die entsprechend gewählten elektromagnetischen Wellen, in diesem Fall für den sichtbaren und den nahen Infrarot-Bereich. Da das Behandeln mit der Diffusionsfarbe ein relativ einfacher Prozess ist, kann der erfindungsgemäße Effekt ohne nennenswerten Mehraufwand erreicht werden.

Vorzugsweise ist das Glas bzw. das Grundglas ein Borosilikatglas. Borosilikatgläser zeichnen sich durch eine besonders hohe Inertie und Beständigkeit gegenüber Chemikalien, so dass aus dem Borosilikatglas keine unerwünschten Substanzen in die pharmazeutische Substanz migrieren. Zudem lässt sich Borosilikatglas gut sterilisieren, ist gasdicht und temperaturbeständig.

Die Borosilikatgläser können folgende Anteile in Gewichtsprozent umfassen:

| | |
|---|---|
| SiO₂: | 70% bis 82% |
| B₂O₃: | 7% bis 13% |
| ∑ Na₂O + K₂O: | 4% bis 8% |
| Al₂O₃: | 2% bis 7% |
| ∑ CaO + MgO: | 0 % bis 5 % |

Hierbei ist zu erwähnen, dass die Anzahl an Komponenten relativ gering ist, so dass eine gute Aussage auf das Verhalten der pharmazeutischen Substanz gegenüber ermöglicht wird. Das Borosilikatglas kann dotiert werden. Da aber die Dotierungen anteilsmäßig so gering sind, werden insbesondere die chemischen und mechanischen Eigenschaften nicht geändert. Die angegebenen Anteile des Borosilikatglases lassen Dotierungen zu.

Vorzugsweise bestehen der erste und/oder der zweite Verbindungskörper aus Sinterglas. Der Verbindungskörper lässt sich sehr kostengünstig aus Sinterglas herstellen, der zudem allein aufgrund der Glaspartikel eine erhöhte Strahlungsabsorption für die entsprechend gewählten elektromagnetischen Wellen aufweist.

Der erste und/oder der zweite Verbindungskörper können sich chemisch und/oder strukturell vom Grundkörper und/oder vom ersten oder zweiten Anschlusskörper unterscheiden. Die chemische Zusammensetzung und/oder die Struktur werden an den gewünschten Stellen so geändert, dass die Strahlungsabsorption für elektromagnetische Wellen erhöht wird. Die Anschlusskörper und der Grundkörper können dabei unverändert bleiben, so dass der Aufwand zur Fertigung des erfindungsgemäßen Behälters besonders niedrig gehalten werden kann. Der Verbindungskörper kann hierzu mit einer Diffusionsfarbe behandelt sein, so dass sich die Strahlungsabsorption aufgrund der oben näher beschriebenen Effekte auf einfache Weise erhöhen lässt.

Der erste und/oder der zweite Verbindungskörper können aus einem Glaspulver oder einer Glaspaste bestehen oder diese/s umfassen. Unter Glaspaste wird dabei mit Flüssigkeit gebundenes Glaspulver verstanden. In dieser Ausgestaltung weist der Verbindungskörper ähnliche Eigenschaften wie in dem Fall auf, in welchem er aus Sinterglas besteht oder dieses umfasst. Aufgrund der höheren Strahlenabsorption schmilzt das Glaspulver auf, wodurch die Anschlusskörper und der Grundkörper miteinander verbunden werden. Bei der Glaspaste verdampft beim Bestrahlen die Flüssigkeit, so dass das Glaspulver übrigbleibt.

Zudem betrifft die Erfindung ein Verfahren zum Herstellen eines Behälters zum Lagern und/oder Applizieren einer pharmazeutischen Substanz insbesondere nach einem der zuvor beschriebenen Ausführungsbeispiele, umfassend folgende Schritte:
- Bereitstellen eines Grundkörpers aus Glas, der eine im Wesentlichen hohlzylindrische Form aufweist und einen Hohlraum umschließt, wobei der Grundkörper ein erstes Ende mit einer ersten Öffnung aufweist,
- Bereitstellen eines ersten Anschlusskörpers aus Glas, der einen Durchtrittskanal umfasst,
- Verbinden des ersten Anschlusskörpers am Grundkörper in einem ersten Anschlussbereich, in welchem der Behälter eine oder mehrere erste Absorptionszonen aufweist, in welcher der Behälter eine höhere Strahlungsabsorption für elektromagnetische Wellen in einem vorbestimmten Wellenlängenbereich aufweist als der Grundkörper außerhalb des ersten Anschlussbereichs, wobei das Verbinden durch Bestrahlen zumindest des ersten Anschlussbereichs mit elektromagnetischen Wellen im vorbestimmten Wellenlängenbereich erfolgt, wodurch der Behälter durch erhöhtes Absorbieren der elektromagnetischen Wellen im ersten Anschlussbereich stärker erwärmt wird als außerhalb des ersten Anschlussbereichs und der erste Anschlusskörpers so mit dem Grundkörper verbunden wird, dass der Durchtrittskanal mit der ersten Öffnung kommuniziert.

Die Vorteile, welche sich mit dem erfindungsgemäßen Verfahren erzielen lassen, entsprechen denjenigen, die für den betreffenden Behälter genannt worden sind. Zusammenfassend sei an dieser Stelle gesagt, dass der Herstellungsprozess dadurch vereinfacht werden kann, dass der erste Anschlusskörper entsprechend vorgefertigt und anschließend auf die oben genannte Weise mit dem Grundkörper verbunden werden kann. Mehrstufige Umformschritte, die exakt aufeinander abgestimmt sein müssen, entfallen, so dass der erfindungsgemäße Behälter auf eine kostengünstigere und einfachere Weise als im Stand der Technik bereitgestellt werden kann.

Das erfindungsgemäße Verfahren wird durch folgende Schritte weitergebildet:
- Anordnen eines ersten Verbindungskörpers aus Glas im ersten Anschlussbereich zwischen dem Grundkörper und dem ersten Anschlusskörper, und
- Verbinden des ersten Verbindungskörpers mit dem ersten Anschlusskörper und dem Grundkörper durch Bestrahlen zumindest der ersten Absorptionszone mit elektromagnetischen Wellen im vorbestimmten Wellenlängenbereich.

Neben den für die zuvor genannten Ausführungsbeispiele beschriebenen Vorteilen lässt sich der erfindungsgemäße Behälter mit diesem Verfahren deshalb besonders einfach und kostengünstig herstellen, da nur der Verbindungskörper eine erhöhte Strahlungsabsorption für elektromagnetische Wellen aufweisen muss. Sowohl der Anschlusskörper als auch der Grundkörper können unverändert bleiben.

Das erfindungsgemäße Verfahren wird durch folgende Schritte weitergebildet:
- Bereitstellen des Grundkörpers aus Glas, der ein zweites Ende mit einer zweiten Öffnung aufweist,
- Bereitstellen eines zweiten Anschlusskörpers,
- Verbinden des zweiten Anschlusskörpers am Grundkörper in einem zweiten Anschlussbereich, in welchem der Behälter eine zweite Absorptionszone aufweist, in welcher der Behälter eine höhere Strahlungsabsorption für elektromagnetische Wellen aufweist als der Grundkörper außerhalb der zweiten Absorptionszone, wobei das Verbinden durch Bestrahlen zumindest der zweiten Absorptionszone mit elektromagnetischen Wellen im vorbestimmten Wellenlängenbereich erfolgt, wodurch der Behälter durch erhöhtes Absorbieren der elektromagnetischen Wellen in der zweiten Absorptionszone stärker erwärmt wird als außerhalb der zweiten Absorptionszone.

Der auf diese Weise hergestellte Behälter eignet sich insbesondere, um als vorgefüllte Spritze zum Applizieren der pharmazeutischen Substanz verwendet zu werden.

Das erfindungsgemäße Verfahren wird durch folgende Schritte weitergebildet:
- Anordnen eines zweiten Verbindungskörpers aus Glas im zweiten Anschlussbereich zwischen dem Grundkörper und dem zweiten Anschlusskörper, und
- Verbinden des zweiten Verbindungskörpers mit dem zweiten Anschlusskörper und dem Grundkörper durch Bestrahlen zumindest der zweiten Absorptionszone mit elektromagnetischen Wellen.

Ein mit diesem Verfahren hergestellter Behälter eignet sich insbesondere, um Durchmesser- und Formunterschiede zwischen dem Grundkörper und dem Anschlusskörper zu überbrücken.

Das erfindungsgemäße Verfahren, bei dem der Grundkörper am ersten Ende oder im Bereich des ersten Endes und/oder am zweiten Ende oder im Bereich des zweiten Endes eine Fügefläche und der erste und/oder der zweite Anschlusskörper eine Gegenfügefläche aufweisen, an denen der Grundkörper mit dem ersten und/oder dem zweiten Anschlusskörper verbunden ist, wird durch folgenden Schritt weitergebildet:
- Aufrauen der Fügefläche und/oder der Gegenfügefläche, bevor die Schritte des Anordnens und des Bestrahlens vorgenommen werden.

Hierdurch wird ein ähnlicher Effekt wie bei Sinterglas erreicht, so dass auch hier die Strahlungsabsorption für entsprechend gewählte elektromagnetische Wellen erhöht wird, ohne dass zusätzlich eine Dotierung eingebracht werden muss. Hierdurch wird der Vorteil erreicht, dass am fertigen Behälter keine Spuren mehr zurückbleiben, welche auf die aufgerauten Fügeflächen und/oder Gegenfügeflächen hindeuten, so dass ein besonders homogener Behälter bereitgestellt werden kann.

Verfahrensschritte, die für die Herstellung des Behälters ohne separate Verbindungskörper beschrieben worden sind, können ebenfalls bei der Herstellung des Behälters mit einem Verbindungskörper angewendet werden.

Die Anschlusskörper können aus einem Glastropfen mittels Pressen, aus Rohrabschnitten oder Glasplatten mittels Heißumformung, aus Glaspulver mittels Lasersintern (Rapid-Prototyping-Verfahren) oder mittels eines keramischen 3D-Drucks mit anschließendem Sintern hergestellt werden. Werden die Anschlusskörper aus Glastropfen, Rohrabschnitten oder aus Glasplatten hergestellt, so erfolgt die Erhöhung der Strahlungsabsorption für elektromagnetische Wellen vorzugsweise durch Dotierung, Aufrauen oder durch die Verwendung von Diffusionsfarben. Werden die Anschlusskörper durch Sintern hergestellt, kann auf das Dotieren, Aufrauen oder die Verwendung von Diffusionsfarben verzichtet werden, da die Strahlungsabsorption bereits schon durch die Streuung an den Partikeln des Sinterglases ausreichend erhöht werden kann.

Dabei ist es besonders bevorzugt, wenn der Grundkörper, der erste Anschlusskörper, der zweite Anschlusskörper, der erste Verbindungskörper und/oder der zweite Verbindungskörper aus demselben Grundglas bestehen. Insbesondere kann hierdurch die Lagerhaltung vereinfacht werden, da nur ein Grundglas beschafft und vorgehalten werden muss.

Weiterhin kann der Behälter in der oder den ersten Absorptionszonen und/oder der oder den zweiten Absorptionszonen aus Sinterglas bestehen. Mit der Verwendung von Sinterglas lässt sich die Strahlungsabsorption für elektromagnetische Strahlung auf relativ einfache Weise erhöhen. Weitere Maßnahmen zum Erhöhen der Strahlungsabsorption müssen nicht ergriffen werden. Zudem lassen sich mit der Verwendung von Sinterglas auch komplexere Geometrien fertigen, die mit normalem Glas nicht zu fertigen wären.

Des Weiteren betrifft die Erfindung die Verwendung eines Behälters nach einem der zuvor beschriebenen Ausführungsbeispiele zum Lagern und/oder Applizieren einer pharmazeutischen Substanz.

Die vorliegende Erfindung wird anhand von bevorzugten Ausführungsbeispielen unter Bezugnahme auf die anhängenden Zeichnungen im Detail erläutert. Es zeigen
- Figur 1: ein erstes Ausführungsbeispiel eines erfindungsgemäßen Behälters im nicht-verbundenen Zustand,
- Figur 2: ein zweites Ausführungsbeispiel des erfindungsgemäßen Behälters im nicht-verbundenen Zustand,
- Figur 3: eine prinzipielle Darstellung eines Verfahrens zum Herstellen des Behälters gemäß dem ersten Ausführungsbeispiel, und
- Figur 4: eine prinzipielle Darstellung eines Verfahrens zum Herstellen des Behälters gemäß dem zweiten Ausführungsbeispiel.

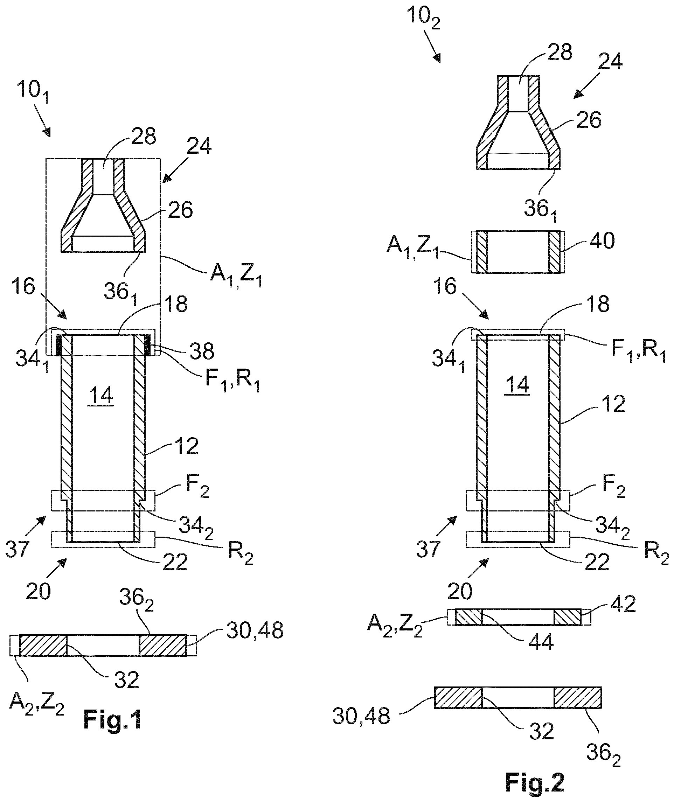

In Figur 1 ist ein erstes Ausführungsbeispiel eines erfindungsgemäßen Behälters 10₁ in einem noch nicht fertig montierten Zustand gezeigt. Der Behälter 10₁ umfasst einen Grundkörper 12, der eine im Wesentlichen hohlzylindrische Form aufweist und einen Hohlraum 14 umschließt. Somit weist der Grundkörper 12 ein erstes Ende 16, welches eine erste Öffnung 18 umschließt, sowie ein zweites Ende 20 auf, welches eine zweite Öffnung 22 umschließt.

Ferner umfasst der erfindungsgemäße Behälter 10₁ einen ersten Anschlusskörper 24, der in einem ersten Anschlussbereich A₁ mit dem Grundkörper 12 verbunden ist. Im vorliegenden Fall ist der erste Anschlussbereich A₁ so definiert, dass er einen Bereich des ersten Endes R₁ des Grundkörpers 12 umschließt und sich von diesem über den gesamten Anschlusskörper 24 erstreckt. Der erste Anschlusskörper 24 weist einen konusförmigen Abschnitt 26 sowie einen Durchtrittskanal 28 auf. Der erste Anschlusskörper 24 kann nicht näher dargestellte Anschlussgeometrien aufweisen, beispielsweise einen Luer-Lock-Anschluss zum Anschließen einer Kanüle oder eines Schlauches.

Weiterhin weist der Behälter 10₁ einen zweiten Anschlusskörper 30 auf, der in etwa ringförmig ausgestaltet ist und eine Durchgangsöffnung 32 aufweist, die in ihrem Durchmesser in etwa dem Außendurchmesser des Grundkörpers 12 in einem Absatz 37 entspricht. In diesem Fall erstreckt sich ein zweiter Anschlussbereich A₂ nur auf den zweiten Anschlusskörper 30.

Zudem weist der Grundkörper 12 eine erste Fügefläche 34₁ und eine zweite Fügefläche 34₂ auf, die jeweils mit einer ersten Gegenfügefläche 36₁ des ersten Anschlusskörpers 24 und einer zweiten Gegenfügefläche 36₂ des zweiten Anschlusskörpers 30 zusammenwirken, wie später noch genauer erklärt werden wird. Im dargestellten Beispiel ist die zweite Fügefläche 34₂ im Absatz 37 des Grundkörpers 12 angeordnet.

Im Bereich F₁ der ersten Fügefläche 34₁ unterscheidet sich der Grundkörper 12 derart vom übrigen Bereich, dass der Grundkörper 12 im Bereich F₁ der ersten Fügefläche 34₁ eine erhöhte Strahlungsabsorption für elektromagnetische Wellen aufweist. Hierzu kann der Grundkörper 12 an der ersten Fügefläche 34₁ aufgeraut sein. Alternativ ist der Grundkörper 12 an der ersten Fügefläche 34₁ aufgeraut und im Bereich R₁ des ersten Endes 16, welche in diesem Fall mit dem Bereich F₁ der ersten Fügefläche 34₁ zusammen fällt, mit einer Diffusionsfarbe 38 behandelt worden. In einer weiteren Alternative ist der Grundkörper 12 im Bereich R₁ des ersten Endes 16, welche in diesem Fall mit dem Bereich F₁ der ersten Fügefläche 34₁ zusammen fällt, mit einer Diffusionsfarbe 38 behandelt worden, ohne dass die Fügefläche 34₁ aufgeraut ist. Die beiden letzten Alternativen sind in Figur 1 dargestellt.

Unter den jeweiligen Bereichen F₁, R₁ sollen Bereiche verstanden werden, die in jedem Fall das erste Ende 16 bzw. die erste Fügefläche 34₁, zusätzlich aber einen in der Größe wählbaren Bereich des Grundkörpers 12 umfassen.

Der erste Anschlusskörper 24 weist im dargestellten Beispiel insgesamt eine höhere Strahlungsabsorption für elektromagnetische Wellen auf, beispielsweise dadurch, dass er aus einen Sinterglas hergestellt ist. Er muss somit keine zusätzliche Dotierung enthalten sondern kann allein auf Grund der erhöhten Streuung schon höher strahlungsabsorbierend sein. Folglich weist der Behälter 10₁ im ersten Anschlussbereich A₁ eine erste Absorptionszone Z₁ auf, die in diesem Fall den ersten Anschlusskörper 24 und Bereich des ersten Endes R₁ des Grundkörpers umfasst und somit mit dem mit dem ersten Anschlussbereich A₁ zusammenfällt. Folglich soll unter einer Absorptionszone Z₁ alle Bereiche innerhalb des Anschlussbereichs A₁ verstanden werden, die eine erhöhte Absorption für einen vorbestimmten Wellenlängenbereich λ aufweisen.

Allerdings ist es genauso gut möglich, nur einen die Gegenfügefläche 36₁ umfassenden Teil des ersten Anschlusskörpers 24 aus Sinterglas zu fertigen, so dass nur dieser Teil eine erhöhte Strahlungsabsorption aufweist. In diesem Fall erstreckt sich die erste Absorptionszone Z₁ über den Bereich des ersten Endes R₁ des Grundkörpers 12 und nur teilweise über den Anschlusskörper 24, so dass die Absorptionszone Z₁ nicht mit dem Anschlussbereich A₁ zusammenfällt, sondern nur Teil hiervon ist.

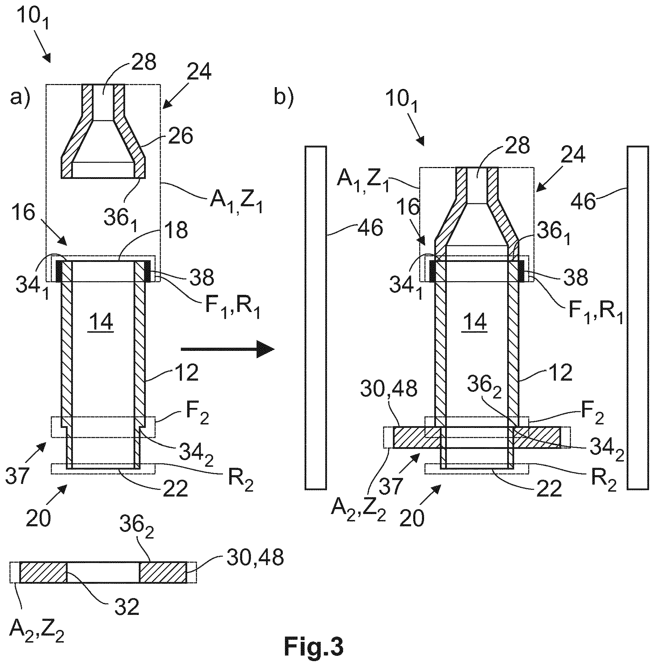

Der zweite Anschlusskörper 30 kann in diesem dargestellten Beispiel ebenfalls aus Sinterglas hergestellt sein. Der Bereich R₂ des zweiten Endes 20 kann je nach Gestaltung des Grundkörpers 12 den Bereich F₂ der zweiten Fügefläche 34₂ umfassen, wobei die beiden Bereiche F₂, R₂ nicht gleich groß sein müssen. Da der zweite Anschlusskörper 30 aus Sinterglas besteht und damit eine höhere Strahlungsabsorption für elektromagnetische Wellen aufweist, ist es nicht notwendig, den Grundkörper 12 im Bereich der zweiten Fügefläche 34₂ oder im Bereich R₂ des zweiten Endes 20 gesondert zu gestalten. In diesem Fall weist der Behälter 10₁ einen zweiten Anschlussbereich A₂ auf, der sich nur über den zweiten Anschlusskörper 30 erstreckt, nicht aber den Bereich des zweiten Endes R₂ einschließt (vgl. Figur 3b)).

Entsprechend erstreckt sich eine zweite Absorptionszone Z₂ über den zweiten Anschlusskörper 30 und fällt mit dem zweiten Anschlussbereich A₂ zusammen.

Alternativ kann der Grundkörper 12 im Bereich F₂ der zweiten Fügefläche 34₂ oder im Bereich R₂ des zweiten Endes 20 auf dieselbe Art und Weise aufgebaut sein wie im Bereich F₁ der ersten Fügefläche 34₁ bzw. im Bereich R₁ des ersten Endes 16. In diesem Fall umfasst der zweite Anschlussbereich A₂ wie auch die zweite Absorptionszone Z₂ noch den Bereich F₂, aber ebenfalls nicht den Bereich des zweiten Endes R₂.

Sowohl der erste Anschlusskörper 24 als auch der zweite Anschlusskörper 30 und der Grundkörper 12 bestehen aus demselben Grundglas, insbesondere aus einem Borosilikatglas.

In Figur 2 ist ein zweites Ausführungsbeispiel des erfindungsgemäßen Behälters 10₂ ebenfalls in nicht-montierten Zustand gezeigt, der im Wesentlichen dem ersten Ausführungsbeispiel 10₁ entspricht. Zusätzlich weist jedoch der Behälter 10₂ gemäß dem zweiten Ausführungsbeispiel einen ersten Verbindungskörper 40 auf, der am ersten Ende 16 zwischen dem Grundkörper 12 und dem ersten Anschlusskörper 24 angeordnet ist. Weiterhin umfasst der Behälter 10₂ einen zweiten Verbindungskörper 42, der in etwa ringförmig aufgebaut ist und eine Durchgangsöffnung 44 aufweist, deren Durchmesser dem Außendurchmesser des Grundkörpers 12 entspricht. Im dargestellten Beispiel weisen nur der erste und der zweite Verbindungskörper 40, 42 eine erhöhte Strahlungsabsorption auf, wohingegen der erste und der zweite Anschlusskörper 24, 30 sowie der Grundkörper 12 keine Behandlung erfahren haben, die eine Erhöhung der Strahlungsabsorption zur Folge hat. Folglich weist der Behälter 10₂ einen ersten Anschlussbereich A₁ auf, der sich über den ersten Verbindungskörper 40 erstreckt und mit der erste Absorptionszone Z1 zusammenfällt. Weiterhin weist der Behälter 10₂ einen zweiten Anschlussbereich A₂ auf, der sich über den zweiten Verbindungskörper 42 erstreckt und mit der zweiten Absorptionszone Z₂ zusammenfällt.

In Figur 3 ist ein Verfahren zum Herstellen des Behälters 10₁ gemäß dem ersten Ausführungsbeispiel anhand von Prinzipskizzen dargestellt. Ausgehend vom nicht-montierten Zustand, der in Figur 3a) dargestellt ist, wird der erste Anschlusskörper 24 am ersten Ende 16 so am Grundkörper 12 positioniert, so dass die erste Fügefläche 34₁ mit der ersten Gegenfügefläche 36₁ in Kontakt tritt. Der zweite Anschlusskörper 30 wird über das zweite Ende 20 auf den Grundkörper 12 aufgeschoben, bis die zweite Gegenfügefläche 36₂ im Absatz 37 an der zweiten Fügefläche 34₂ anliegt. Anschließend wird der Behälter 10₁ in einem nicht näher dargestellten Aggregat mit elektromagnetischen Wellen einer vorbestimmten Wellenlänge λ bestrahlt, wozu eine Strahlungsquelle 46 vorgesehen ist (siehe Figur 3b)). Je nach verwendeter Strahlungsquelle 46 kann dabei ein Wellenlängenbereich λ verwendet werden. Infolge der Bestrahlung erwärmt sich der Behälter 10₁ in der ersten Absorptionszone Z₁ stärker als außerhalb der ersten Absorptionszone Z₁. Im dargestellten Beispiel erwärmt sich der erste Anschlusskörper 24 stärker, da er aus Sinterglas ist. Weiterhin erwärmt sich der Grundkörper 12 im Bereich R₁ des ersten Endes 16 stärker, da er dort mit der Diffusionsfarbe 38 beschichtet ist. Der Anschlusskörper 24 und der Bereich R₁ des ersten Endes bilden zusammen den ersten Anschlussbereich A₁, der mit der ersten Absorptionszone Z₁ zusammenfällt. Die Diffusionsfarbe 38 kann so ausgestaltet sein, dass sich Silberkomplexe in den oberflächennahen Schichten des Grundkörpers 12 bilden. Auch wenn nur in den oberflächennahen Schichten eine erhöhte Strahlungsabsorption vorliegt und zunächst nur diese Schichten aufgrund der Bestrahlung erhitzt werden, so erhitzt sich der Grundkörper 12 durch Wärmeleitung im gesamten Bereich R₁ des ersten Endes 16 stärker als im übrigen Bereich.

Am zweiten Ende 20 erwärmt sich nur der zweite Anschlusskörper 30 stärker, da auch er aus Sinterglas hergestellt ist. Der Grundkörper 12 ist bezüglich einer erhöhten Strahlungsabsorption nicht besonders behandelt, so dass er nicht stärker erhitzt wird. Daher fallen der zweite Anschlussbereich A₁ und die zweite Absorptionszone Z₂ zusammen.

Die Strahlungsquelle 46 wird so betrieben, dass der erste und der zweite Anschlusskörper 24, 30 sowie der Bereich R₁ des ersten Endes 16 auf eine Temperatur oberhalb des Transformationspunktes T_{G}, insbesondere oberhalb des Erweichungspunktes EW, erwärmt werden. Die übrigen Bereiche heizen sich nur auf Temperaturen auf, die unterhalb des Erweichungspunktes EW liegen, aber im Bereich des Transformationspunktes T_{G} liegen können. Folglich verringert sich die Viskosität der beiden Anschlusskörper 24, 30 insgesamt und des Grundkörpers 12 im Bereich R₁ des ersten Endes 16 durch die Strahlung und der Grundkörper 12 zusätzlich durch Wärmeleitung im Bereich F₂ der zweiten Fügefläche 34₂, so dass sich infolgedessen ein Stoffschluss zwischen dem Grundkörper 12 und den Anschlusskörpern 24, 30 ausbildet. Beim Abkühlen entsteht eine hermetisch dichte Verbindung. Da die übrigen Bereiche des Grundkörpers 12 infolge der Bestrahlung auf Temperaturen unterhalb des Erweichungspunktes EW, insbesondere unterhalb oder im Bereich des Transformationspunktes T_{G} erwärmt werden, deformiert er sich nicht und bleibt formstabil. Es kann eine thermische Nachbehandlung erfolgen, um Spannungen im Behälter 10₁ abzubauen. Da aber der Behälter 10₁ nicht nur im Bereich der Verbindungsstelle zwischen dem Grundkörper 12 und den Anschlusskörpern 24, 30 erwärmt wird, halten sich die Spannungen in Grenzen. Zudem muss die Strahlungsquelle 46 nicht gesondert ausgerichtet werden, was die Gestaltung des Aggregates vereinfacht.

Zusätzlich kann der Behälter 10₁ vor und/oder während der Behandlung mit der Strahlungsquelle 46 vorgewärmt werden, um die Temperaturunterschiede zwischen den einzelnen Bauteilen 12, 24, 30 so gering zu halten, dass es nicht zu hohen thermischen Spannungen kommt, welche die Bauteile oder den entstehenden Verbund 10₁ zerstören können.

Im verbundenen Zustand wirkt der zweite Anschlusskörper 30 als ein Fingerflansch 48, so dass der nun fertige Behälter 10₁ als eine vorfüllbare Spritze zum Lagern und Applizieren einer pharmazeutischen Substanz verwendet werden kann.

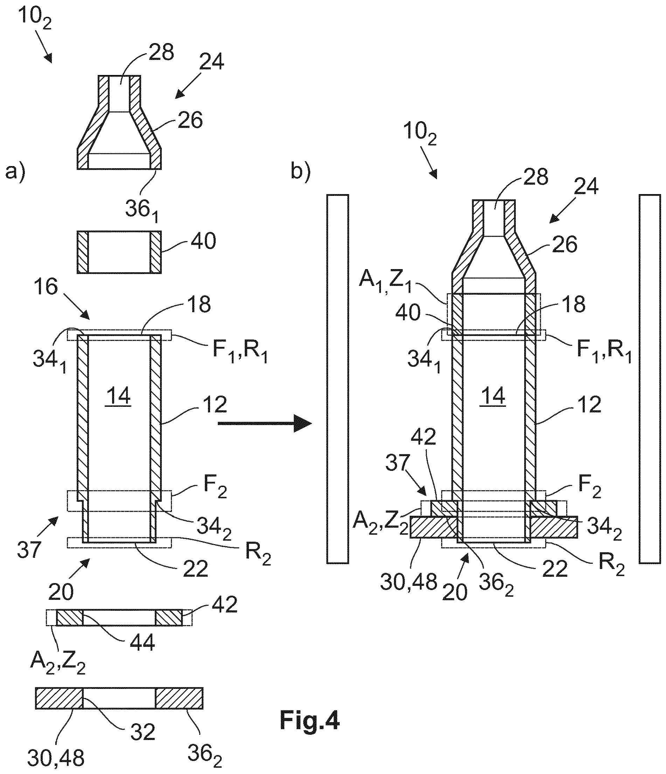

In Figur 4 ist eine prinzipielle Darstellung eines Verfahrens zum Herstellen des Behälters 10₂ gemäß dem zweiten Ausführungsbeispiel widergegeben. Die Herstellung des Behälters 10₁ nach dem zweiten Ausführungsbeispiel geschieht im Wesentlichen auf dieselbe Weise wie die Herstellung des Behälters 10₁ nach dem ersten Ausführungsbeispiel mit der Ausnahme, dass zwischen dem Grundkörper 12 und dem ersten und dem zweiten Anschlusskörper 24, 30 der erste bzw. zweite Verbindungskörper 40, 42 platziert werden. Im dargestellten Beispiel sollen nur die beiden Verbindungskörper 40, 42 eine erhöhte Strahlungsabsorption aufweisen, so dass diese auf eine Temperatur, die über dem Transformationspunkt T_{G}, insbesondere oberhalb des Erweichungspunktes EW, liegen, erhitzt werden, schmelzen und folglich eine stoffschlüssige Verbindung mit dem Grundkörper 12 und dem ersten Anschlusskörper 24 bzw. dem zweiten Anschlusskörper 30 eingehen. Dabei werden der Grundkörper 12 und der erste und zweite Anschlusskörper 24, 30 auf eine Temperatur unter dem Erweichungspunktes EW, aber im Bereich des Transformationspunkte T_{G} erwärmt, so dass sie sich nicht deformieren. Beim Abkühlen entsteht eine hermetisch dichte Verbindung. Es kann eine thermische Nachbehandlung erfolgen, um Spannungen im Behälter 10₁ abzubauen. Im verbundenen Zustand wirkt der zweite Anschlusskörper 30 als ein Fingerflansch 48, so dass der nun fertige Behälter 10₂ als eine vorfüllbare Spritze zum Lagern und Applizieren einer pharmazeutischen Substanz verwendet werden kann.

Bevorzugte Strahlungsquellen für die Erzeugung von elektromagnetischen Wellen umfassen jeweils eine oder mehrere UV-Strahlungsquellen, beispielsweise Quecksilberdampflampen und/oder Strahlungsquellen, die im sichtbaren Bereich abstrahlen, beispielsweise Xenon-Kurzbogen-Hochdrucklampen und/oder Infrarot-Strahlungsquellen, insbesondere Infrarot-Strahlungsquellen, die kurzwellige Infrarotstrahlung abgeben, beispielsweise Nd:YAG-Laser, Diodenlaser oder Wolfram-IR-Strahler und/oder Mikrowellenstrahlungsquellen, beispielsweise Magnetrons. Kurzwellige Infrarotstrahlung (kIR-Strahlung) hat sich hier als besonders geeignet herausgestellt, die von Wolfram-Halogen-IR-Strahlern mit 1500 bis 3500K Farbtemperatur erzeugt wird. Bei dieser Heiztechnologie wird die Erwärmung nicht allein durch die Temperatur des Aggregats, sondern im Wesentlichen durch die IR-Strahlung der Heizelemente und das Absorptionsverhalten des aufzuheizenden Körpers bestimmt.

### Erstes Ausführungsbeispiel (Wolfram-Halogen-IR-Strahler)

Ausgangspunkt ist eine Anordnung wie in Figur 4 gezeigt, bestehend aus einem Grundkörper 12 aus einem Borosilikat-Rohrglas mit 45 mm Gesamtlänge und einem Außendurchmesser von 8 mm sowie zwei Anschlusskörpern 24, 30 aus Sinterglas, ebenfalls aus demselben Borosilikatglas, die mit 5 % Fe₂O₃ dotiert sind. Der Grundkörper 12 und die Anschlusskörper 24, 30 werden wie gezeigt angeordnet und mit einer Geschwindigkeit von 1 cm/s bis 10 cm/s durch einen Durchlaufofen gefahren. In Höhe der Verbindungskörper 40, 42 wird von außen mit Wolfram-Halogen-IR-Strahlern als Strahlungsquelle 46 mit einer Farbtemperatur von 1500 bis 3000 K auf den Behälter 10₂ gestrahlt. Die Infrarot-Strahlungsleistung wird so eingestellt, dass die Anschlusskörper 24, 30 innerhalb von 1 bis 60 sec anschmelzen und sich mit dem Grundkörper 12 hermetisch dicht verbinden. Eine konventionelle Zusatzheizung mit 500 W elektrischer Leistung oder eine Infrarotheizung oder eine andere geeignete Heizeinrichtung erwärmt während der Infrarotbestrahlung den gesamten Behälter 10₂ auf mehrere 100°C soweit, dass durch die lokale Infrarotbestrahlung keine unzulässig hohen Spannungen im Grundkörper 12 oder in den Anschlusskörpern 24, 30 entstehen können. Nach erfolgreicher Verschmelzung wird eine weitere thermische Nachbehandlung angeschlossen, um die restlichen Spannungen aus dem nun fertigen Behälter 10₂ zu entfernen.

### Zweites Ausführungsbeispiel (Laser)

Ausgangspunkt ist eine Anordnung wie in Figur 4 gezeigt, bestehend aus einem Grundkörper 12 aus einem Borosilikat-Rohrglas mit 45 mm Gesamtlänge und einem Außendurchmesser von 8 mm sowie zwei Anschlusskörpern 24, 30 aus Sinterglas, ebenfalls aus demselben Borosilikatglas, die mit 5% Fe₂O₃ dotiert sind. Der Grundkörper 12 und die Anschlusskörper 24, 30 werden senkrecht auf einem Rotationsteller fixiert und mit einer Umdrehungsgeschwindigkeit von 1 bis 120 U/min rotiert. In Höhe der Verbindungskörper 40, 42 wird radial von außen mit einem Laserstrahl und einer Wellenlänge zwischen 900 bis 1500 nm auf die Anschlusskörper 24, 30 gestrahlt. Dabei dient ein geeignetes Gerät zur Strahlungsausweitung, so dass eine Laserlinie von etwa 4 mm Länge entsteht. Die Laserleistung wird so eingestellt, dass die Verbindungskörper 40, 42 innerhalb von 1 bis 60 sec aufschmelzen und den Grundkörper 12 mit den Anschlusskörpern 24, 30 hermetisch dicht verbinden. Eine konventionelle Zusatzheizung mit 500 W elektrischer Leistung oder eine Infrarotheizung oder eine andere geeignete Heizeinrichtung erwärmt während der Infrarotbestrahlung den gesamten Behälter 10₂ auf mehrere 100°C soweit, dass durch die lokale Infrarotbestrahlung keine unzulässig hohen Spannungen im Grundkörper 12 oder in den Anschlusskörpern entstehen können. Nach erfolgreicher Verschmelzung wird eine weitere thermische Nachbehandlung angeschlossen, um die restlichen Spannungen aus dem nun fertigen Behälter 10₂ zu entfernen.

### Drittes Ausführungsbeispiel (Mikrowellenresonator)

Ausgangspunkt ist eine Anordnung wie in Figur 4 gezeigt, bestehend aus einem Grundkörper 12 aus einem Borosilikat-Rohrglas mit 45 mm Gesamtlänge und einem Außendurchmesser von 8 mm sowie zwei Anschlusskörpern 24, 30 aus Sinterglas, ebenfalls aus demselben Borosilikatglas, die mit 1 bis 90% Fe gefüllt sind. Der Grundkörper 12 und die Anschlusskörper 24, 30 werden senkrecht auf einem Rotationsteller fixiert und mit einer Umdrehungsgeschwindigkeit von 1 bis 120 U/min in einem zylindrischen Mikrowellenresonator mit einem Innendurchmesser von 30 mm rotiert, wobei mittels eines Mikrowellenhohlleiters Mikrowellenstrahlung mit einer Frequenz von 0,9 bis 30 GHz in den Mikrowellenresonator eingekoppelt werden. Die Leistung des Mikrowellenresonators wird durch Pulsen oder andere geeignete Regelungsmaßnahmen so eingestellt, dass die Verbindungskörper 40, 42 innerhalb von 1-60 sec aufschmelzen und den Grundkörper 12 mit den Anschlusskörpern 24, 30 hermetisch dicht verbinden. Eine konventionelle Zusatzheizung mit 500 W elektrischer Leistung oder eine Infrarotheizung oder eine andere geeignete Heizeinrichtung erwärmt während der Infrarotbestrahlung den gesamten Behälter 10₁ auf mehrere 100°C soweit, dass durch die lokale Infrarotbestrahlung keine unzulässig hohen Spannungen im Grundkörper 12 oder in den Anschlusskörpern 24, 30 entstehen können. Nach erfolgreicher Verschmelzung wird eine weitere thermische Nachbehandlung angeschlossen, um die restlichen Spannungen aus dem nun fertigen Behälter 10₁ zu entfernen.

### Bezugszeichenliste

- 10, 10₁, 10₂: Behälter
- 12: Grundkörper
- 14: Hohlraum
- 16: erstes Ende
- 18: erste Öffnung
- 20: zweites Ende
- 22: zweite Öffnung
- 24: erster Anschlusskörper
- 26: konusförmiger Abschnitt
- 28: Durchtrittskanal

- 30: zweiter Anschlusskörper
- 32: Durchgangsöffnung
- 34, 34₁, 34₂: Fügefläche
- 36, 36₁, 36₂: Gegenfügefläche
- 37: Absatz
- 38: Diffusionsfarbe
- 40: erster Verbindungskörper
- 42: zweiter Verbindungskörper
- 44: Durchgangsöffnung
- 46: Strahlungsquelle
- 48: Fingerflansch

- A₁: erster Anschlussbereich
- A₂: zweiter Anschlussbereich
- F₁: Bereich der ersten Fügefläche
- F₂: Bereich der zweiten Fügefläche
- R₁: Bereich des ersten Endes
- R₂: Bereich des zweiten Endes
- Z₁: erste Absorptionszone
- Z₂: zweite Absorptionszone

## Patentansprüche

1. Behälter zum Lagern und/oder Applizieren einer pharmazeutischen Substanz, umfassend
- einen Grundkörper aus Glas (12), der eine im Wesentlichen hohlzylindrische Form aufweist und einen Hohlraum (14) umschließt, wobei der Grundkörper (12) ein erstes Ende (16) mit einer ersten Öffnung (18) aufweist, und
- einen ersten Anschlusskörper (24) aus Glas, wobei der erste Anschlusskörper (24) einen mit der ersten Öffnung (18) kommunizierenden Durchtrittskanal (28) aufweist,
- der erste Anschlusskörper (24) in einem ersten Anschlussbereich (A₁) mit dem Grundkörper (12) verbunden ist und der Behälter (10) im ersten Anschlussbereich (A₁) eine oder mehrere erste Absorptionszonen (Z₁) aufweist, in welcher der Behälter (10) eine höhere Strahlungsabsorption für elektromagnetische Wellen in einem vorbestimmten Wellenlängenbereich (λ) aufweist als der Grundkörper (12) außerhalb der ersten Absorptionszone (Z₁).

2. Behälter nach Anspruch 1,
**dadurch gekennzeichnet, dass** im ersten Anschlussbereich (A₁) ein erster Verbindungskörper (40) aus Glas angeordnet ist, über welchen der erste Anschlusskörper (24) mit dem Grundkörper (12) verbunden ist.

3. Behälter nach Anspruch 2,
**dadurch gekennzeichnet, dass** die erste Absorptionszone (Z₁) auf den ersten Verbindungskörper (40) begrenzt ist.

4. Behälter nach einem der vorherigen Ansprüche,
**dadurch gekennzeichnet, dass**
- der Behälter einen zweiten Anschlusskörper (30) aus Glas umfasst und
- der Grundkörper (12) ein zweites Ende (20) mit einer zweiten Öffnung (22) aufweist, wobei
- der zweite Anschlusskörper (30) in einem zweiten Anschlussbereich (A₂) mit dem Grundkörper (12) verbunden ist und der Behälter (10) im zweiten Anschlussbereich (A₂) eine oder mehrere zweite Absorptionszonen (Z₂) aufweist, in welcher der Behälter (10) zumindest abschnittsweise eine höhere Strahlungsabsorption für elektromagnetische Wellen in einem vorbestimmten Wellenlängenbereich (λ) aufweist als der Grundkörper (12) außerhalb der zweiten Absorptionszone (Z₂).

5. Behälter nach einem der vorherigen Ansprüche,
**dadurch gekennzeichnet, dass** im zweiten Anschlussbereich (A₂) ein zweiter Verbindungskörper (42) aus Glas angeordnet ist, über welchen der zweite Anschlusskörper (30) mit dem Grundkörper (12) verbunden ist.

6. Behälter nach einem der vorherigen Ansprüche,
**dadurch gekennzeichnet, dass** der Grundkörper (12), der erste Anschlusskörper (24), der zweite Anschlusskörper (30), der erste Verbindungskörper (40) und/oder der zweite Verbindungskörper (42) aus demselben Grundglas bestehen.

7. Behälter nach einem der vorherigen Ansprüche,
**dadurch gekennzeichnet, dass** der Behälter in der oder den ersten Absorptionszonen (Z₁) und/oder der oder den zweiten Absorptionszonen (Z₂) aus Sinterglas besteht.

8. Behälter nach einem der vorherigen Ansprüche,
**dadurch gekennzeichnet, dass** der Behälter in der oder den ersten Absorptionszonen (Z₁) und/oder der oder den zweiten Absorptionszonen (Z₂) zum Erhöhen der Strahlungsabsorption für elektromagnetische Wellen dotiert ist.

9. Behälter nach einem der vorherigen Ansprüche,
**dadurch gekennzeichnet, dass** der Behälter (10) in der oder den ersten Absorptionszonen (Z₁) und/oder in der oder den zweiten Absorptionszonen (Z₂) mit einer Diffusionsfarbe (38) behandelt ist.

10. Verfahren zum Herstellen eines Behälters zum Lagern und/oder Applizieren einer pharmazeutischen Substanz nach einem der vorherigen Ansprüche, umfassend folgende Schritte:
- Bereitstellen eines Grundkörpers (12) aus Glas, der eine im Wesentlichen hohlzylindrische Form aufweist und einen Hohlraum (14) umschließt, wobei der Grundkörper (12) ein erstes Ende (16) mit einer ersten Öffnung (18) aufweist,
- Bereitstellen eines ersten Anschlusskörpers (24) aus Glas, der einen Durchtrittskanal (28) umfasst,
- Verbinden des ersten Anschlusskörpers (24) am Grundkörper (12) in einem ersten Anschlussbereich (A₁), in welchem der Behälter (10) eine oder mehrere erste Absorptionszonen (Z₁) aufweist, in welcher der Behälter (10) eine höhere Strahlungsabsorption für elektromagnetische Wellen in einem vorbestimmten Wellenlängenbereich (λ) aufweist als der Grundkörper (12) außerhalb der ersten Absorptionszone (Z₁), wobei das Verbinden durch Bestrahlen zumindest der ersten Absorptionszone (Z₁) mit elektromagnetischen Wellen in dem vorbestimmten Wellenlängenbereich (λ) erfolgt, wodurch der Behälter (10) durch erhöhtes Absorbieren der elektromagnetischen Wellen in der ersten Absorptionszone (Z₁) stärker erwärmt wird als außerhalb der ersten Absorptionszone (Z₁) und der erste Anschlusskörper (24) so mit dem Grundkörper (12) verbunden wird, dass der Durchtrittskanal (28) mit der ersten Öffnung (18) kommuniziert.

11. Verfahren nach Anspruch 10, umfassend folgende Schritte:
- Anordnen eines ersten Verbindungskörpers (40) aus Glas im ersten Anschlussbereich (A₁) zwischen dem Grundkörper (12) und dem ersten Anschlusskörper (24), und
- Verbinden des ersten Verbindungskörpers (40) mit dem ersten Anschlusskörper (24) und dem Grundkörper (12) durch Bestrahlen zumindest der ersten Absorptionszone (Z₁) mit elektromagnetischen Wellen in dem vorbestimmten Wellenlängenbereich (λ).

12. Verfahren nach Anspruch 10 oder 11, umfassend folgende Schritte:
- Bereitstellen des Grundkörpers (12) aus Glas, der ein zweites Ende (20) mit einer zweiten Öffnung (22) aufweist,
- Bereitstellen eines zweiten Anschlusskörpers (30) aus Glas, ,
- Verbinden des zweiten Anschlusskörpers (30) am Grundkörper (12) in einem zweiten Anschlussbereich (A₂), in welchem der Behälter (10) eine zweite Absorptionszone (Z₂) aufweist, in welcher der Behälter (10) eine höhere Strahlungsabsorption für elektromagnetische Wellen in einem vorbestimmten Wellenlängenbereich (λ) aufweist als der Grundkörper (12) außerhalb der zweiten Absorptionszone (Z₂), wobei das Verbinden durch Bestrahlen zumindest der zweiten Absorptionszone (Z₂) mit elektromagnetischen Wellen in dem vorbestimmten Wellenlängenbereich erfolgt, wodurch der Behälter (10) durch erhöhtes Absorbieren der elektromagnetischen Wellen in der zweiten Absorptionszone (Z₂) stärker erwärmt wird als außerhalb der zweiten Absorptionszone (Z₂).

13. Verfahren nach einem der Ansprüche 10 bis 12, umfassend folgende Schritte:
- Anordnen eines zweiten Verbindungskörpers (42) aus Glas im zweiten Anschlussbereich (A₂) zwischen dem Grundkörper (12) und dem zweiten Anschlusskörper (30), und
- Verbinden des zweiten Verbindungskörpers (42) mit dem zweiten Anschlusskörper (30) und dem Grundkörper (12) durch Bestrahlen zumindest der zweiten Absorptionszone (Z₂) mit elektromagnetischen Wellen.

14. Verfahren nach einem der Ansprüche 10 bis 13,
**dadurch gekennzeichnet, dass** der Grundkörper (12), der erste Anschlusskörper (24), der zweite Anschlusskörper (30), der erste Verbindungskörper (40) und/oder der zweite Verbindungskörper (42) aus demselben Grundglas bestehen.

15. Verfahren nach einem der Ansprüche bis 14,
**dadurch gekennzeichnet, dass** der Behälter (10) in der oder den ersten Absorptionszonen (Z₁) und/oder der oder den zweiten Absorptionszonen (Z₂) aus Sinterglas besteht.

## Claims

1. A container for storing and/or applying a pharmaceutical substance, comprising
- a base body made of glass (12), having an essentially hollow cylindrical shape and surrounding a cavity (14), wherein the base body (12) has a first end (16) with a first opening (18), and
- a first connected body (24) made of glass, wherein the first connected body (24) has a passage channel (28) communicating with the first opening (18),
- the first connected body (24) is connected in a first connecting region (A₁) with the base body (12) and the container (10) in the first connecting region (A₁) has one or more first absorption zones (Z₁), in which the container (10) exhibits a higher radiation absorption for electromagnetic waves in a predetermined wavelength range (λ) than the base body (12) outside of the first absorption zone (Z₁).

2. The container according to claim 1, **characterised in that** in the first connecting region (A₁) a first joining body (40) made of glass is disposed, via which the first connected body (24) is connected to the base body (12).

3. The container according to claim 2, **characterised in that** the first absorption zone (Z₁) is delimited on the first joining body (40).

4. The container according to one of the preceding claims, **characterised in that**
- the container comprises a second connected body (30) made of glass and
- the base body (12) has a second end (20) with a second opening (22) wherein
- the second connected body (30) is connected in a second connecting region (A₂) with the base body (12) and the container (10) in the second connecting region (A₂) has one or more second absorption zones (Z₂), in which the container (10) at least in sections exhibits a higher radiation absorption for electromagnetic waves in a predetermined wavelength range (λ) than the base body (12) outside of the second absorption zone (Z₂).

5. The container according to one of the preceding claims, **characterised in that** in the second connecting region (A₂) a second joining body (42) made of glass is disposed, via which the second connected body (30) is connected to the base body (12).

6. The container according to one of the preceding claims, **characterised in that** the base body (12), the first connected body (24), the second connected body (30), the first joining body (40) and/or the second joining body (42) consist of the same glass type.

7. The container according to one of the preceding claims, **characterised in that** the container in the absorption zone or the first absorption zones (Z₁) and/or the absorption zone or the second absorption zones (Z₂) consists of sintered glass.

8. The container according to one of the preceding claims, **characterised in that** the container in the absorption zone or the first absorption zones (Z₁) and/or the absorption zone or the second absorption zones (Z₂) is doped to increase the radiation absorption for electromagnetic waves.

9. The container according to one of the preceding claims, **characterised in that** the container (10) in the absorption zone or the first absorption zones (Z₁) and/or the absorption zone or the second absorption zones (Z₂) is treated with a diffusion colour (38).

10. A method for producing a container for storing and/or applying a pharmaceutical substance according to one of the preceding claims, comprising the following steps:
- Providing a base body made of glass (12), having an essentially hollow cylindrical shape and surrounding a cavity (14), wherein the base body (12) has a first end (16) with a first opening (18), and
- Providing a first connected body (24) made of glass, which comprises a passage channel (28),
- Connecting the first connected body (24) to the base body (12) in a first connecting region (A₁), in which the container (10) has one or more first absorption zones (Z₁) in which the container (10) exhibits a higher radiation absorption for electromagnetic waves in a predetermined wavelength range (λ) than the base body (12) outside of the first absorption zone (Z₁), wherein the connection takes place by irradiating at least the first absorption zone (Z₁) with electromagnetic waves in the predetermined wavelength range (λ), as a result of which the container (10), due to higher absorption of the electromagnetic waves in the first absorption zone (Z₁) is heated more strongly than outside of the first absorption zone (Z₁) and the first connecting element (24) becomes connected with the base body (12) in such a way that the passage channel (28) communicates with the first opening (18).

11. The method according to claim 10, comprising the following steps:
Arranging a first joining body (40) in the first connecting region (A₁) between the base body (12) and the first connecting element (24), and
- Connecting the first joining body (40) with the first connected body (24) and the base body (12) by irradiating at least the first absorption zone (Z₁) with electromagnetic waves in the predetermined wavelength range (λ).

12. The method according to claim 10 or 11, comprising the following steps:
- Providing the glass base body (12), which has a second end (20) with a second opening (22),
- Providing a second connected body (30) made of glass,
- Connecting the second connected body (30) to the base body (12) in a second connecting region (A₂), in which the container (10) has a second absorption zone (Z₂) in which the container (10) exhibits a higher radiation absorption for electromagnetic waves in a predetermined wavelength range (λ) than the base body (12) outside of the second absorption zone (Z₂), wherein the connection takes place by irradiating at least the second absorption zone (Z₂) with electromagnetic waves in the predetermined wavelength range, as a result of which the container (10), due to higher absorption of the electromagnetic waves in the second absorption zone (Z₂), is more strongly heated than outside of the second absorption zone (Z₂).

13. The method according to one of claims 10 to 12, comprising the following steps:
Arranging a second joining body (42) in the second connecting region (A₂) between the base body (12) and the second connected body (30), and
- Connecting the second joining body (42) with the second connected body (30) and the base body (12) by irradiating at least the second absorption zone (Z₂) with electromagnetic waves.

14. The method according to one of claims 10 to 13, **characterised in that** the base body (12), the first connected body (24), the second connected body (30), the first joining body (40) and/or the second joining body (42) consist of the same glass type.

15. The method according to one of claims to 14, **characterised in that** the container (10) in the first absorption zone or the first absorption zones (Z₁) and/ or the second absorption zone or the second absorption zones (Z₂) consists of sintered glass.

## Revendications

1. Récipient destiné à stocker et/ou à appliquer une substance pharmaceutique, comprenant
- un corps de base en verre (12), qui présente une forme essentiellement cylindrique creuse et renferme une cavité (14), le corps de base (12) présentant une première extrémité (16) avec une première ouverture (18) et
- un premier corps de raccordement (24) en verre, le premier corps de raccordement (24) présentant un canal de passage (28) en communication avec la première ouverture (18),
- le premier corps de raccordement (24) est relié, dans une première zone de raccordement (A₁) avec le corps de base (12) et le récipient (10) présente, dans la première zone de raccordement (A₁), une ou plusieurs premières zones d'absorption (Z₁), dans lesquelles le récipient (10) présente une absorption de rayonnement pour les ondes électromagnétiques dans une plage de longueurs d'ondes prédéfinies (λ) supérieure à celle du corps de base (12) en dehors de la première zone d'absorption (Z₁).

2. Récipient selon la revendication 1, **caractérisé en ce qu'**un premier corps de liaison (40) en verre est disposé dans la première zone de raccordement (A₁), par le biais duquel le premier corps de raccordement (24) est relié au corps de base (12).

3. Récipient selon la revendication 2, **caractérisé en ce que** la première zone d'absorption (Z₁) est limitée par le premier corps de liaison (40).

4. Récipient selon une des revendications précédentes, **caractérisé en ce que**
- le récipient comprend un second corps de raccordement (30) en verre et
- le corps de base (12) présente une seconde extrémité (20) comportant une seconde ouverture (22),
- le second corps de raccordement (30) est relié, dans une seconde zone de raccordement (A₂) avec le corps de base (12) et le récipient (10) présente, dans la seconde zone de raccordement (A₂), une ou plusieurs secondes zones d'absorption (Z₂), dans lesquelles le récipient (10) présente au moins par endroits une absorption de rayonnement pour les ondes électromagnétiques dans une plage de longueurs d'ondes prédéfinies (λ) supérieure à celle du corps de base (12) en dehors de la seconde zone d'absorption (Z₂).

5. Récipient selon une des revendications précédentes, **caractérisé en ce qu'**un second corps de liaison (42) en verre est disposé dans la seconde zone de raccordement (A₂), par le biais duquel le second corps de raccordement (30) est relié au corps de base (12).

6. Récipient selon une des revendications précédentes, **caractérisé en ce que** le corps de base (12), le premier corps de raccordement (24), le second corps de raccordement (30), le premier corps de liaison (40) et/ou le second corps de liaison (42) sont constitués du même verre de base.

7. Récipient selon une des revendications précédentes, **caractérisé en ce que** le récipient, dans la ou les premières zones d'absorption (Z₁) et/ou dans la ou les secondes zones d'absorption (Z₂), est constitué de verre fritté.

8. Récipient selon une des revendications précédentes, **caractérisé en ce que** le récipient, dans la ou les premières zones d'absorption (Z₁) et/ou dans la ou les secondes zones d'absorption (Z₂), est dopé pour augmenter l'absorption du rayonnement pour les ondes électromagnétiques.

9. Récipient selon une des revendications précédentes, **caractérisé en ce que** le récipient (10), dans la ou les premières zones d'absorption (Z₁) et/ou dans la ou les secondes zones d'absorption (Z₂), est traité avec une couleur de diffusion.

10. Procédé de fabrication d'un récipient destiné à stocker et/ou à appliquer une substance pharmaceutique selon une des revendications précédentes, comprenant les étapes suivantes :
- fourniture d'un corps de base (12) en verre, qui présente une forme essentiellement cylindrique creuse et renferme une cavité (14), le corps de base (12) présentant une première extrémité (16) avec une première ouverture (18),
- fourniture d'un premier corps de raccordement (24) en verre, qui comprend un canal de passage (28),
- liaison du premier corps de raccordement (24) avec le corps de base (12) dans une première zone de raccordement (A₁), dans laquelle le récipient (10) présente une ou plusieurs premières zones d'absorption (Z₁), dans lesquelles le récipient (10) présente une absorption du rayonnement pour les ondes électromagnétiques dans une plage de longueurs d'ondes prédéfinie (λ) supérieure à celle du corps de base (12) en dehors de la première zone d'absorption (Z₁), la liaison étant effectuée par irradiation au moins de la première zone d'absorption (Z₁) avec des ondes électromagnétiques dans la plage de longueurs d'ondes prédéfinie (λ), moyennant quoi le récipient (10) est réchauffé plus fortement par une absorption accrue des ondes électromagnétiques dans la première zone d'absorption (Z₁) qu'en dehors de la première zone d'absorption (Z₁) et le premier corps de raccordement (24) est ainsi relié au corps de base (12), de telle sorte que le canal de passage (28) soit en communication avec la première ouverture (18).

11. Procédé selon la revendication 10, comprenant les étapes suivantes :
- disposition d'un premier corps de liaison (40) en verre dans la première zone de raccordement (A₁) entre le corps de base (12) et le premier corps de raccordement (24) et
- liaison du premier corps de liaison (40) avec le premier corps de raccordement (24) et le corps de base (12) par irradiation, au moins, de la première zone d'absorption (Z₁) avec des ondes électromagnétiques dans la plage de longueurs d'ondes prédéfinie (λ).

12. Procédé selon la revendication 10 ou 11, comprenant les étapes suivantes :
- fourniture du corps de base (12) en verre, présentant une seconde extrémité (20) avec une seconde ouverture (22),
- fourniture d'un second corps de raccordement (30) en verre,
- liaison du second corps de raccordement (30) avec le corps de base (12) dans une seconde zone de raccordement (A₂), dans laquelle le récipient (10) présente une seconde zone d'absorption (Z₂), dans laquelle le récipient (10) présente une absorption du rayonnement pour les ondes électromagnétiques dans une plage de longueurs d'ondes prédéfinie (λ) supérieure à celle du corps de base (12) en dehors de la seconde zone d'absorption (Z₂), la liaison étant effectuée par irradiation au moins de la seconde zone d'absorption (Z₂) avec des ondes électromagnétiques dans la plage de longueurs d'ondes prédéfinie (), moyennant quoi le récipient (10) est réchauffé plus fortement par une absorption accrue des ondes électromagnétiques dans la seconde zone d'absorption (Z₂) qu'en dehors de la seconde zone d'absorption (Z₂).

13. Procédé selon une des revendications 10 à 12, comprenant les étapes suivantes :
- disposition d'un second corps de liaison (42) en verre dans la seconde zone de raccordement (A₂) entre le corps de base (12) et le second corps de raccordement (30) et
- liaison du second corps de liaison (42) avec le second corps de raccordement (30) et le corps de base (12) par irradiation, au moins, de la seconde zone d'absorption (Z₂) avec des ondes électromagnétiques.

14. Procédé selon une des revendications 10 à 13, **caractérisé en ce que** le corps de base (12), le premier corps de raccordement (24), le second corps de raccordement (30), le premier corps de liaison (40) et/ou le second corps de liaison (42) sont constitués du même verre de base.

15. Procédé selon une des revendications à 14, **caractérisé en ce que** le récipient (10) dans la ou les premières zones d'absorption (Z₁) et/ou dans la ou les secondes zones d'absorption (Z₂), est constitué de verre fritté.
